# FASCICULE DE BREVET EUROPEEN

(11) **EP 3 771 343 B1**
(45) Date de publication et mention de la délivrance du brevet: **03.09.2025**
(21) Numéro de dépôt: 20188730.4
(22) Date de dépôt: 30.07.2020
(51) Int. Cl.: A61K 36/185, B01D 11/02, B30B 9/26, B30B 15/32, B30B 9/06, A23L 5/30

(54) **PROCÉDÉ ET DISPOSITIF D'OBTENTION D'UN MACÉRÂT VÉGÉTAL AQUEUX LIQUIDE**
VERFAHREN UND VORRICHTUNG ZUR HERSTELLUNG EINES FLÜSSIGEN WÄSSRIGEN PFLANZENMAZERATS
METHOD AND DEVICE FOR OBTAINING AN AQUEOUS LIQUID PLANT MACERATION

(30) Priorité: 31.07.2019 FR 1908785
(43) Date de publication de la demande: 03.02.2021
(73) Titulaire: Laboratoires Arkopharma, 06510 Carros (FR); Avignon Universite, 84029 Avignon Cedex 1 (FR)
(72) Inventeur: CHEMAT, Farid, 84310 Morières les Avignon (FR); FATTORINI, Dominique, 06100 Nice (FR); IMBERT, Rémi, 06610 La Gaude (FR)
(74) Mandataire: Murgitroyd & Company

(56) Documents cités:
- CN-A- 102 764 207
- CN-A- 107 212 411
- FR-A1- 2 718 459
- MOULTON AND L C WANG K J: "A Pilot-Plant Study of Continuous Ultrasonic Extraction of Soybean Protein", vol. 47, no. 4, July 1982 (1982-07-01), pages 1127 - 1129, XP002714168, ISSN: 0022-1147, Retrieved from the Internet <URL:http://onlinelibrary.wiley.com/doi/10.1111/j.1365-2621.1982.tb07632.x/pdf> [retrieved on 20131003], DOI: 10.1111/J.1365-2621.1982.TB07632.X
- "Enhancing Extraction Processes in the Food Industry", vol. 20114942, 26 September 2011, CRC PRESS, ISBN: 978-1-4398-4595-0, ISSN: 2155-336X, article FARID LEBOVKA ET AL: "Ultrasonically Assisted Diffusion Processes", pages: 123 - 144, XP055700787, DOI: 10.1201/b11241-5
- "Enhancing Extraction Processes in the Food Industry", vol. 20114942, 26 September 2011, CRC PRESS, ISBN: 978-1-4398-4595-0, ISSN: 2155-336X, article FARID LEBOVKA ET AL: "Combined Extraction Techniques", pages: 173 - 194, XP055700793, DOI: 10.1201/b11241-7
- "Solids and Liquids Separation", 1995, article LOUISE FERRANTE: "Solids and Liquids Separation", XP055701179

## Description

### Domaine technique

L'invention concerne le domaine de l'extraction végétale et plus particulièrement le domaine des macérâts végétaux aqueux, à savoir obtenus par macération d'un substrat végétal dans l'eau.

### Etat de la technique

Dans le domaine de l'extraction à l'échelle industrielle, et en particulier de l'extraction végétale, les procédés conventionnels d'extraction présentent bon nombre d'inconvénients majeurs, comme la récupération insuffisante des composés bioactifs (polyphénols, flavonoïdes, terpènes etc.), la durée très longue des extractions, l'utilisation de solvants organiques d'origine naturelle ou pétrolière, accompagnées d'un chauffage intensif donnant lieu, entre autres, à des consommations énergétiques élevées et induisant possiblement la dégradation de tout ou partie des composés bioactifs d'intérêt (en particulier des composés bioactifs thermosensibles). A titre d'exemple, les plantes médicinales sont traditionnellement extraites soit en utilisant l'hexane comme solvant (pour extraire les métabolites lipophiles) ou bien par un mélange hydro-alcoolique contenant une forte proportion d'alcool éthylique afin d'extraire les composés hydrophiles. Le solvant est généralement porté à ébullition pendant plusieurs heures, suivi d'une évaporation dudit solvant pour une formulation ultérieure.

Les principales limites de ces procédés conventionnels d'extraction végétale résident en des durées d'extraction longues (tel qu'indiqué précédemment) et en l'évaporation de grandes quantités de solvants organiques. Comme cela est connu de l'homme du métier, cette importante évaporation induit en particulier des risques sanitaires et/ou environnementaux. Par ailleurs, des traces de solvant organique demeurent au sein de l'extrait à formuler (ce qui nécessite des étapes de purification additionnelle en vue d'éliminer ce solvant organique) mais également dans les plantes épuisées, lesquelles doivent être traitées comme des déchets chimiques, ce qui représente, là encore, un inconvénient notable. Aucun solvant organique n'est inoffensif. Ils ont tous des effets sur la santé, variables selon les produits et la nature de l'exposition professionnelle : une exposition unique à forte dose (effets aigus) ou des expositions répétées. Il convient donc, dans la mesure du possible, de s'affranchir de l'utilisation de tels solvants organiques et d'éviter la présence de traces de solvant(s) organique(s) dans le macérât final (et, de préférence, également dans les plantes épuisées postérieurement au procédé d'extraction).

Par ailleurs, la forte sélectivité du solvant organique d'extraction ne permet pas d'extraire une grande variété de composés bioactifs (polyphénols, flavonoïdes, terpènes etc.) du substrat végétal, ce qui est généralement souhaitable dans le cas de formulations à visée nutritionnelle (compléments alimentaires) ou pharmaceutique. Eu égard aux problématiques inhérentes à l'emploi de solvants d'extraction organique, il pourrait être envisagé d'utiliser l'eau comme solvant pour l'extraction des composés bioactifs d'un substrat végétal. Toutefois, l'homme du métier sait qu'une telle utilisation serait immanquablement limitée par la mauvaise diffusion de l'eau dans le tissu végétal à cause de sa viscosité, de sa mouillabilité et de sa polarité.

Dans le cadre de l'amélioration constante des procédés d'extraction végétale, plusieurs procédés d'extraction végétale « intensifiés » ont été mis au point avec pour objectif des extractions plus rapides, une gestion plus maîtrisée et réfléchie de l'énergie utilisée, une augmentation du transfert de masse et de chaleur, une réduction ou élimination des solvants dits organiques, une réduction ou élimination des déchets, une taille d'équipement plus adaptée ainsi qu'une réduction des étapes unitaires. On trouve notamment l'extraction assistée par micro-ondes, l'extraction assistée par les champs électriques pulsés, l'extraction par les fluides supercritiques, l'extraction par les liquides sous pression et l'extraction assistée par ultrasons. Concernant cette dernière, il convient de noter que les ultrasons sont généralement appliqués à l'extraction de produits naturels pour améliorer les rendements ou les cinétiques d'extraction. La cavitation ultrasonore est utilisée pour générer des micros jets de liquide à proximité des particules de matrice végétale. Les micros jets de liquide font des impacts sur la matrice et les parois cellulaires. Les particules sont abrasées et les membranes cellulaires sont perforées, libérant et accélérant les transferts de composés. Plusieurs demandes de brevet font état de l'utilisation des ultrasons pour l'extraction végétale au moyen d'un solvant naturel à base d'huile végétale comme les demandes de brevet publiées sous les numéros de référence WO 2010/112760 et WO 2013/117634.

Bien que l'extraction assistée par ultrasons de produits naturels soit connue, il reste des limitations techniques comme l'utilisation d'une seule plante et la spécificité du dispositif ultrasonore qui ne peut être effectif que pour une certaine famille de plantes et aussi des limitations de l'extraction due aux problèmes de préparation de la matière première, de mélange au sein de l'extracteur et du post-traitement qui doit être adapté avant formulation ou utilisation.

Les demandes de brevet FR2718459 et WO2014/152785 décrivent des procédés d'extraction assistée par ultrasons afin d'extraire des huiles végétales. Dans la mesure où la diffusion des ultrasons au sein des corps oléagineux s'avère problématique, des émulsions huile/eau sont vraisemblablement créées dans les procédés décrits au sein de FR2718459 et WO2014/152785 dans l'optique d'améliorer le passage des ultrasons. La formation - artificielle - de ces émulsions impose des étapes de traitement additionnelles en fin de procédé afin de « désémulsionner » l'émulsion (à savoir séparer l'huile de l'eau au sein de ladite émulsion). Ces étapes additionnelles représentent notamment l'inconvénient d'induire des coûts et délais de traitement supplémentaires.

La demande de brevet US2010/0303974, quant à elle, divulgue un procédé d'extraction comprenant une étape de prétraitement relative au séchage et au broyage du végétal ainsi qu'une étape d'extraction assistée par un dispositif à ultrasons. Une étape de centrifugation est réalisée postérieurement à l'étape d'extraction sur de petits volumes de matière végétale et uniquement à des fins d'analyse (cf. tableau 1). Le procédé divulgué dans US2010/0303974 a uniquement vocation à être mis en œuvre au sein d'un laboratoire de recherche et n'est en aucun cas industrialisable.

La demande de brevet CN102764207 A enseigne notamment un procédé de préparation d'un extrait concentré de thé Pu'Erh (thé de Chine issu d'une tradition très ancienne) en vue de la préparation d'un dentifrice à base de thé Pu'Erh. Ledit procédé est décrit comme comprenant notamment une étape de lixiviation (« leaching », en langue anglaise), de feuilles de thé Pu'Erh - préalablement écrasées et broyées - sous ultrasons, à une température comprise entre 50°C et 80°C, suivie d'une étape de refroidissement de l'extrait ainsi obtenu (pour atteindre la température ambiante), d'une étape de séparation solide-liquide réalisée par filtration sous pression (par exemple au moyen d'un filtre-presse), et d'une pluralité d'étapes de centrifugation successives (de deux à quinze), chacune réalisée durant une période de temps comprise entre 5 et 25 minutes et à une vitesse comprise entre 4500 et 8000 rpm. L'obligation de procéder au retrait du surnageant à l'issue de chacune des opérations de centrifugation induit un risque quant à la robustesse/reproductibilité du procédé de CN102764207 A, ainsi qu'une difficulté pour la maîtrise des pertes lors des opérations de centrifugation successives. En outre, et notamment en raison du rapport en poids solide/liquide important mis en œuvre dans le réacteur utilisé aux fins d'une extraction par lixiviation (en général de l'ordre de 1/2) et du fait que le corps solide est immobile, la cavitation ultrasonore rencontre très probablement des difficultés à atteindre l'intérieur du corps solide (en l'espèce des feuilles de thé Pu'Erh préalablement écrasées et broyées), induisant possiblement une détexturation insuffisante dudit corps solide et donnant vraisemblablement lieu in fine à une baisse du rendement d'extraction. A toutes fins utiles, il est rappelé que la « lixiviation » (ou plus communément « percolation »), est une technique d'extraction solide-liquide consistant à faire couler un liquide (solvant) à travers un corps solide immobile, généralement à plusieurs reprises, afin d'extraire les composés bioactifs dudit corps solide (la percolation du solvant par gravité permettant le lavage et l'extraction de composés bioactifs).

CN 107 212 411 décrit un procédé d'extraction d'une poudre de maca à l'aide d'un transducteur ultrasonique.

De même, MOULTON AND L C WANG K J: "A Pilot-Plant Study of Continuous Ultrasonic Extraction of Soybean Protein", décrit un procédé d'extraction ultrasonique de proteines de soja.

Farid Lebovka ET AL: "Ultrasonically Assisted Diffusion Processes" illustre les connaissances sur l'extraction dans le domaine alimentaire et notamment les avantages d'une extraction avec l'aide d'ultrasons.

A la lumière de ce qui précède, il s'avère donc nécessaire d'améliorer les procédés et dispositifs d'extraction végétale existants afin de surmonter tout ou partie des inconvénients listés supra. En particulier, il apparaît qu'un défi majeur en matière d'extraction végétale (concernant l'industrie pharmaceutique et celle des compléments alimentaires), consiste à mettre au point un procédé et un dispositif correspondant fiables et efficaces permettant l'obtention de macérâts végétaux liquides contenant une grande variété de composés bioactifs (de préférence de composés bioactifs hydrophiles), directement incorporables dans des formulations ou conditionnements (ou directement consommables), tout en s'affranchissant de l'utilisation de solvant(s) organique(s) (et donc sans traces de solvant(s) organique(s) dans le macérât liquide final et également avantageusement dans les plantes épuisées postérieurement au procédé d'extraction) et d'un chauffage intensif (afin de préserver les composés bioactifs thermosensibles).

### Exposé de l'invention

Dans le cadre de leurs recherches, les inventeurs ont découvert que tout ou partie de ces objectifs pouvaient être atteints en mettant en oeuvre le procédé selon l'invention, à savoir un procédé d'obtention d'un macérât végétal aqueux liquide, ledit procédé comprenant (de préférence successivement ou consécutivement) les étapes suivantes :
a) fourniture d'un substrat végétal,
b) macération dudit substrat végétal dans l'eau par macération assistée par ultrasons, jusqu'à l'obtention d'un macérât végétal aqueux,
   ladite macération étant effectuée dans au moins une cuve de macération comprenant en son sein, avantageusement sur sa paroi interne, au moins deux niveaux de transducteur(s) ultrasonique(s) distincts, chaque niveau de transducteur(s) ultrasonique(s) comprenant au moins un, et de préférence au moins deux, transducteurs ultrasoniques,
c) obtention d'un macérât végétal aqueux liquide par séparation du macérât végétal aqueux liquide et du substrat végétal, ladite séparation étant effectuée par pressage du macérât végétal aqueux obtenu à l'étape b), de préférence sous une pression uniforme comprise entre environ 2 bars et environ 5 bars (par exemple entre 2 et 5 bars), préférablement entre environ 2,5 bars et environ 3,5 bars (par exemple entre 2,5 et 3,5 bars), avantageusement d'environ 3 bars (par exemple de 3 bars).

Selon un mode de réalisation préféré, l'étape b) est effectuée partiellement ou entièrement
- de préférence entièrement - sous agitation.

De manière préférée, après l'étape c), le procédé comprend les étapes suivantes :
d) purification, de préférence par centrifugation, du macérât végétal aqueux liquide obtenu à l'étape c) de manière à obtenir un macérât végétal aqueux liquide purifié, avantageusement sous forme de solution aqueuse,
e) récupération du macérât végétal aqueux liquide ainsi purifié.

Selon un mode de réalisation de l'invention, ledit procédé consiste essentiellement en - ou consiste en - les étapes a) - e), préférablement réalisées de manière successive ou consécutive. Avantageusement, les étapes b) - e) sont effectuées successivement, dans un flux continu, sans interruption du procédé (le cas échéant, simplement sous la supervision d'un opérateur). Ceci permet non seulement d'obtenir un procédé parfaitement industrialisable mais également efficace et économe en temps homme/jour.

De manière préférée, le procédé comprend, entre les étapes d) et e), les étapes suivantes :
d1) mesure de la turbidité du macérât végétal aqueux liquide purifié à l'étape d),
d2) comparaison de la turbidité mesurée à l'étape d1) avec un seuil de turbidité prédéterminé,
   - d3) élimination dudit macérât végétal aqueux liquide purifié lorsque la turbidité mesurée à l'étape d1) est supérieure audit seuil de turbidité, ou
   - d3') récupération, à l'étape e), du macérât végétal aqueux liquide purifié lorsque la turbidité mesurée à l'étape d1) est inférieure ou égale audit seuil de turbidité.

Selon un mode de réalisation de l'invention, ledit procédé consiste essentiellement en - ou consiste en - les étapes a), b), c), d), d1), d2), d3) ou d3'), et e), préférablement réalisées de manière successive ou consécutive. Avantageusement, lorsque la turbidité mesurée à l'étape d1) est inférieure ou égale au seuil de turbidité, les étapes b), c), d), d1), d2), d3') et e) sont effectuées successivement, dans un flux continu, sans interruption du procédé (le cas échéant simplement sous la supervision d'un opérateur). Ceci permet non seulement d'obtenir un procédé parfaitement industrialisable mais également efficace et économe en temps homme/jour.

De manière préférée, l'étape d) est au moins une étape de centrifugation, dont les paramètres comprennent de préférence :
- une vitesse de centrifugation comprise entre 3000 et 10000 rpm, préférablement supérieure à 5000 rpm et inférieure ou égale à 10000 rpm, avantageusement comprise entre 5200 et 7500 rpm, de manière particulièrement préférée comprise entre 6000 et 7000 rpm, et/ou
- un débit en macérât végétal aqueux liquide purifié compris entre 5 et 15 m³/h, de préférence entre 7 et 13 m³/h, préférablement entre 8 et 12 m³/h, avantageusement entre 9 et 11 m³/h, de manière préférée d'environ 10 m³/h, avantageusement les deux.

De manière préférée, ladite étape c) comprend au moins deux opérations de pressage, de préférence réalisées de manière successive ou consécutive. Ceci permet d'optimiser le macérât végétal aqueux liquide obtenu (en particulier lorsque lesdites au moins deux opérations de pressage sont réalisées de manière successive ou consécutive), à savoir permet d'obtenir un meilleur rendement d'extraction. De manière particulièrement avantageuse, ladite étape c) comprend au moins deux opérations de pressage successives (ou consiste en deux opérations de pressage successives) du susdit macérât végétal aqueux, la pression exercée sur ledit macérât végétal aqueux lors de la deuxième (ou seconde) opération de pressage étant exercée suivant un axe différent de l'axe suivant lequel la pression est exercée sur le macérât végétal aqueux lors de la première opération de pressage. Ceci permet de séparer le macérât végétal aqueux liquide du substrat végétal avec une efficacité accrue.

De manière préférée, l'étape b) est réalisée à une température de 20°C à 80°C, de préférence de 30°C à 75°C, préférablement de 40°C à 70°C, avantageusement de 55°C à 65°C.

De manière préférée, le procédé comprend avant l'étape b), au moins une étape de prétraitement dudit substrat végétal, telle qu'au moins une étape de séchage et/ou de réduction de taille du substrat végétal, avantageusement les deux.

L'invention concerne également :
- le macérât végétal aqueux liquide directement obtenu par le procédé d'obtention d'un macérât végétal aqueux liquide selon l'invention.

Un autre aspect de l'invention concerne un procédé de conditionnement d'un macérât végétal aqueux liquide dans un contenant à usage pharmaceutique, cosmétique, nutritionnel ou vétérinaire, tel qu'un flacon, un sachet unidose, un stick unidose, une flaconnette (petit flacon), une ampoule bouteille unidose, ou une ampoule (de préférence dans une ampoule, préférablement une ampoule à extrémité(s) cassable(s), avantageusement une ampoule à extrémités cassables telle qu'une ampoule en verre - typiquement ampoule en verre dite « à deux pointes » - par exemple d'une contenance de 10 ou 15 ml), dans lequel ledit macérât végétal aqueux liquide est le macérât végétal aqueux liquide obtenu à l'étape c) ou à l'étape e) (lorsque l'étape e) est présente) du procédé d'obtention d'un macérât végétal aqueux liquide selon l'invention, ledit procédé de conditionnement comprenant l'/les étape(s) suivante(s) :
- le cas échéant procéder à l'ouverture du contenant si celui-ci est obturé,
- remplir, par tout moyen, ledit contenant totalement ou partiellement à l'aide dudit macérât végétal aqueux liquide puis, le cas échéant,
- obturer ledit contenant ainsi rempli, de préférence de manière hermétique.

L'invention concerne également :
- le produit directement obtenu par le procédé de conditionnement d'un macérât végétal aqueux liquide selon l'invention.

Un autre aspect de l'invention concerne un dispositif (ou une installation) d'obtention d'un macérât végétal aqueux liquide, adapté pour mettre en œuvre le procédé d'obtention, ledit dispositif comprenant :
i) au moins une cuve de macération comprenant en son sein, avantageusement sur sa paroi interne, au moins deux niveaux de transducteur(s) ultrasonique(s) distincts, chaque niveau de transducteur(s) ultrasonique(s) comprenant au moins un, et de préférence au moins deux, transducteurs ultrasoniques,
   ladite au moins une cuve de macération comprenant en outre au moins un moyen d'agitation mécanique adapté pour maintenir le substrat végétal à une distance prédéterminée des transducteurs ultrasoniques, ladite au moins une cuve de macération étant adaptée pour recevoir le substrat végétal, l'eau et effectuer l'étape b),
ii) au moins un organe de pressage, tel qu'au moins un pressoir (ou une presse), adapté pour recevoir, à l'issue de l'étape b), le macérât végétal aqueux et effectuer l'étape c),
iii) de préférence au moins une centrifugeuse, préférablement une centrifugeuse à assiettes, adaptée pour recevoir le macérât végétal aqueux liquide obtenu à l'étape c) et effectuer l'étape d), lorsque le procédé comprend ladite étape d),
iv) optionnellement au moins un dispositif de séchage et/ou au moins un dispositif de réduction de taille du substrat végétal (ou au moins un dispositif de séchage et de réduction de taille du substrat végétal), par exemple au moins un dispositif de séchage et au moins un dispositif de réduction de taille du substrat végétal, adapté(s) pour effectuer au moins une étape de séchage et/ou au moins une étape de réduction de taille du substrat végétal (par exemple par fragmentation [cassure, écrasement etc.], ou coupe), avant l'étape b),
v) avantageusement au moins un turbidimètre, adapté pour contrôler la turbidité du macérât végétal aqueux liquide, préférablement obtenu à l'étape d), lorsque le procédé comprend ladite étape d).

De préférence, le dispositif selon l'invention comprend une pompe de vidange connectée à ladite au moins une cuve de macération (plus précisément, en communication de fluide avec ladite au moins une cuve de macération), ladite pompe de vidange étant adaptée pour conduire/transférer, en fin de macération, le macérât végétal aqueux dans ledit au moins un organe de pressage (tout en préservant, dans une certaine mesure, l'homogénéité du macérât végétal aqueux).

Selon un mode de réalisation préféré, ladite au moins une cuve de macération, ledit au moins un organe de pressage, ladite au moins une centrifugeuse (lorsqu'elle est présente) et ledit au moins un turbidimètre (lorsqu'il est présent) sont connectés les uns aux autres par un système (ou ensemble) de conduites (correctement dimensionnées). En d'autres termes, ces éléments sont en communication de fluide. Ceci permet d'éviter les manipulations et/ou interruptions entre lesdits éléments et, par conséquent, de réduire le temps homme/jour tout en gagnant en efficacité, en robustesse et en reproductibilité.

Ledit dispositif comprend au sein de ladite au moins une cuve de macération, avantageusement sur la paroi interne de ladite au moins une cuve de macération, au moins deux niveaux de transducteur(s) ultrasonique(s) distincts (en d'autres termes, chacun desdits au moins deux niveaux de transducteur(s) ultrasonique(s) distincts étant positionné à une hauteur différente au sein de ladite au moins une cuve de macération), de préférence au moins trois niveaux de transducteur(s) ultrasonique(s) distincts (en d'autres termes, chacun desdits au moins trois niveaux de transducteur(s) ultrasonique(s) distincts étant positionné à une hauteur différente au sein de ladite au moins une cuve de macération) ; chaque niveau de transducteur(s) ultrasonique(s) comprenant au moins un, et de préférence au moins deux, transducteur(s) ultrasonique(s). Cet agencement spécifique des transducteurs ultrasoniques au sein de la cuve de macération permet de minimiser le phénomène de rétention ou bourrage lors de la macération ou de la vidange du macérât végétal aqueux. En outre, les transducteurs ultrasoniques sont, de préférence, positionnés sur la paroi interne de ladite au moins une cuve de macération et, avantageusement, sont à fleur de la paroi, ce qui élimine tout risque de zone de rétention du macérât végétal aqueux (en réduisant l'espace transducteur/paroi). En outre, ledit agencement spécifique des transducteurs ultrasoniques au sein de la cuve de macération, couplé au susdit moyen d'agitation mécanique (en particulier lorsque celui-ci est un moyen d'agitation mécanique adapté pour permettre un mouvement descendant ou ascendant du substrat végétal à l'intérieur de la cuve de macération et, ainsi, faciliter le mouillage dudit substrat végétal lors de l'incorporation de celui-ci dans l'eau, tel qu'un moyen d'agitation à pales, comprenant par exemple une ou plusieurs hélices), assure efficacité, robustesse et reproductibilité, et ce quels que soient le type de matériel végétal (par exemple le type de plante, contrairement à bon nombre de dispositifs de l'art antérieur, spécifiques d'un type ou d'une famille de plantes), la ou les partie(s) de plante(s) (contrairement à bon nombre de dispositifs de l'art antérieur, spécifiques d'une partie de plante), et la taille du lot traité au moyen du dispositif selon l'invention. Ces effets techniques avantageux sont encore accrus par la forme spécifique de la cuve de macération comprenant, selon un mode de réalisation préféré, une partie supérieure sensiblement cylindrique (voire cylindrique) et une partie inférieure sensiblement conique (voire conique). Le dispositif selon l'invention, permettant d'obtenir un macérât végétal aqueux liquide, peut donc être légitimement qualifié de dispositif « universel ». Les propriétés avantageuses liées à l'agencement spécifique des transducteurs ultrasoniques au sein de ladite au moins une cuve de macération (cf. supra), avantageusement sur la paroi interne de ladite au moins une cuve de macération, sont optimales pour un agencement à « trois niveaux de transducteur(s) ultrasonique(s) distincts », en particulier lorsque le premier niveau est situé en bas de cuve de macération, le deuxième en milieu de cuve et le troisième en haut de cuve ; les trois niveaux de transducteur(s) ultrasonique(s) distincts susvisés étant préférablement positionnés sensiblement à équidistance les uns des autres (voire à équidistance les uns des autres).

### Définitions

***Macération*** : technique d'extraction solide-liquide qui consiste à laisser au moins un corps solide dans un liquide (solvant) ou un mélange de liquides (solvants), généralement froid, pour en extraire les espèces chimiques. La quantité importante de liquide (solvant) utilisée dans le cadre de la macération rend cette technique d'extraction parfaitement adaptée au couplage avec un traitement par ultrasons, dans la mesure où les ondes ultrasonores se propagent à travers les milieux liquides afin de générer le phénomène de cavitation recherché (contrairement à d'autres techniques d'extraction solide-liquide telles que la « lixiviation » (ou « percolation ») dans lesquelles le ratio solide/liquide (solvant) est plus important). Ce couplage macération/traitement par ultrasons est encore plus efficace lorsque la macération est effectuée sous agitation (exposition accrue du substrat végétal aux micro-jets de liquide induits par la cavitation ultrasonore, donnant notamment lieu à une libération accrue des composés bioactifs par détexturation du substrat végétal et offrant donc un meilleur rendement d'extraction).

***Macérât :*** produit d'une macération. Il est constitué d'un mélange corps solide/solvant(s).

***Macérât végétal:*** produit d'une macération d'un substrat végétal dans un solvant ou un mélange de solvants (solvant(s) de macération). Il est constitué d'un mélange substrat végétal/solvant(s) de macération.

***Macérât végétal aqueux :*** macérât végétal résultant de la macération d'un substrat végétal dans l'eau (l'eau étant le solvant de macération). Il est constitué d'un mélange substrat végétal/eau. Après séparation solide-liquide, il permet d'obtenir d'un côté un macérât végétal aqueux liquide (cf. définition infra) et de l'autre le gâteau de filtrat de plante(s) (cf. également définition infra).

***Macérât végétal aqueux liquide* :** macérât végétal aqueux, sous forme liquide (par exemple sous forme de suspension aqueuse ou de solution aqueuse) obtenu par macération d'un substrat végétal dans l'eau et séparation solide-liquide (par exemple par pressage).

***Macération assistée par ultrasons* :** macération assistée par des ondes ultrasonores qui se propagent à travers les milieux liquides.

***Ultrasons* :** terme employé lorsque l'on se réfère à des ondes vibratoires dont la fréquence est supérieure à la limite maximale d'audibilité de l'oreille humaine (20 kHz). Les ultrasons ne présentent pas de différences physiques avec les sons. Plusieurs effets peuvent être attribués à l'utilisation des ultrasons de puissance tels que le déplacement des molécules de liquide autour de leur point d'équilibre ou les déplacements convectifs appelés vents ultrasonores. Lorsque des ultrasons se propagent à travers un liquide, les oscillations des molécules provoquent la formation de zones de compression et de dépression. En dessous d'un certain seuil caractéristique du liquide considéré, les forces maintenant la cohésion du liquide sont vaincues et les bulles de cavitation contenant la vapeur du liquide et des gaz dissous apparaissent. Ce phénomène, appelé cavitation, a été étudié de manière théorique et expérimentale. Le comportement des bulles dépend de leur taille et de la nature du champ ultrasonore local qui détermine la stabilité ou l'implosion de ces cavités comme indiqué dans le document **[1].**

Il y a plusieurs types de bulles de cavitation. Celles dites transitoires n'existent que pendant quelques cycles acoustiques avant d'imploser violemment. Sachant que la durée de vie de telles bulles est trop faible pour observer un transfert de matière par diffusion de gaz vers l'intérieur ou l'extérieur de la bulle, leur implosion ne se trouve pas amortie et procède d'une grande violence. On peut ainsi atteindre des jets à plus de 120 m/s et des pressions de l'ordre de 1000 atmosphères, ce qui permet d'intensifier les modes de transfert liquide-solide et liquide-liquide que ce soit en transfert de matière, de chaleur ou en quantité de mouvement. Les ultrasons ont des actions mécaniques et physiques, notamment lors de l'explosion des bulles de cavitation. La présence d'un obstacle adjacent provoque de plus la perte de symétrie du système par rapport à une implosion au sein de la solution. Le principal effet physique et mécanique des ultrasons est alors la production de micro-jets dirigés vers une surface solide lors de l'implosion des bulles de cavitation. Ces micro-jets peuvent atteindre des vitesses considérables (plus de 120 m/s) et pourraient avoir une influence prépondérante dans les actions induites par la cavitation et dans l'augmentation de l'agitation à l'interface végétal/solvant comme indiqué dans le document **[2].**

A haute intensité, l'onde ultrasonique génère d'intenses forces de pression et de cisaillement ainsi que des gradients de température à travers le matériel végétal. Ce dernier peut physiquement se rompre. L'énergie ultrasonore résultante peut ainsi améliorer les transferts de chaleur, de matière ou bien de quantité de mouvement. Dans de nombreuses applications, la technologie des ultrasons possède des avantages considérables par rapport aux autres technologies conventionnelles. Les ultrasons améliorent le rendement, les qualités organoleptiques ainsi que les propriétés visuelles du produit alimentaire final destiné à la consommation humaine.

***Substrat végétal*** (également dénommé « matériel végétal ») : s'entend de toute matière végétale (telle que plantes, partie(s) de plantes, fruits, algues, champignons, lichens, ou leurs mélanges), entière ou dont la taille a été réduite si opportun/nécessaire, par exemple par fragmentation (cassure, écrasement etc.), coupe ou pulvérisation. Le substrat végétal peut être utilisé sous forme desséchée ou à l'état frais.

Selon l'invention, le substrat végétal est choisi de préférence parmi le bois, les tiges, les pétales, les feuilles, les parties aériennes, les racines, les rhizomes, les écorces, les fleurs, les graines, les fruits ou leurs mélanges.

Selon un mode de réalisation de l'invention, le substrat végétal est sélectionné parmi la liste suivante *: Harpagophytum procumbens, Harpagophytum zeyheri, Ribes nigrum, Salix alba, Urtica dioica, Urtica urens, Crataegus monogyna, Crataegus laevigata, Crataegus.nigra, Crataegus.pentagyna, Crataegus azarolus, Passiflora incarnata, Citrus aurantium, Lavandula angustifolia, Arctostaphylos uva-ursi, Betula pendula, Betula pubescens, Calluna vulgaris, Hamamelis virginiana, Vitis vinifera, Ruscus aculeatus, Rosmarinus officinalis, Cynara scolymus, Angelica archangelica, Olea europaea L. (avantageusement des feuilles d'olivier),* et leurs mélanges.

***Suspension aqueuse*** : mélange hétérogène dans lequel la phase dispersante est l'eau et dans lequel la phase dispersée est solide, de préférence sous la forme de (quelques) (fines) particules de substrat végétal.

***Solution aqueuse*** : solution dans laquelle une ou plusieurs espèces chimiques (solutés) est/sont dissoute(s) dans l'eau (solvant) pour former un mélange homogène.

***Turbidité*** : Grandeur mesurant le caractère plus ou moins trouble d'un liquide.

***Composé bioactif*** : composé possédant - ou étant susceptible de posséder - une activité pharmacologique, métabolique, immunologique et/ou physiologique chez l'homme ou l'animal.

***Pressage*** : opération de séparation solide-liquide par pression, exercée sur le macérât végétal aqueux (à savoir postérieurement à l'étape de macération), permettant la séparation du macérât végétal aqueux liquide et du substrat végétal épuisé (gâteau de filtrat de plante(s), cf. définition infra). Cette opération permet la récupération du macérât végétal aqueux liquide (par exemple sous forme de suspension aqueuse) non retenue par le substrat végétal.

***Gâteau de filtrat de plante(s)*** : résidu de la plante(s) sous forme solide (humide) correspondant au substrat végétal épuisé. En ce qui concerne la présente invention, il est obtenu par pressage. Tel qu'indiqué précédemment, le gâteau de filtrat de plante(s) obtenu en mettant en œuvre le procédé selon l'invention peut être directement composté, dans la mesure où il est également dépourvu de traces de solvant organique, ce qui s'avère particulièrement avantageux.

***Centrifugeuse (ou séparateur) à assiettes*** : centrifugeuse comprenant des assiettes (ou des disques) et travaillant à des milliers de g (accélération centrifuge) pour permettre une séparation des particules fines de l'extrait liquide (en l'espèce du macérât végétal aqueux liquide) obtenu.

***Détexturation*** : destruction de la paroi végétale et/ou des structures végétales contenant les composés bioactifs.

***Réduction de la taille du substrat végétal (ou « réduction de taille du substrat végétal »)* :** opération - ou ensemble d'opérations - consistant à réduire la taille du substrat végétal d'intérêt. Tel qu'indiqué précédemment, cette réduction peut être obtenue, par exemple, par fragmentation (cassure, écrasement etc.), coupe ou pulvérisation du substrat végétal.

***Niveau*** : Hauteur de quelque chose par rapport à un plan horizontal de référence (conformément aux connaissances générales de l'homme du métier ; cf. https://www.larousse.fr/dictionnaires/francais/niveau/54687). Au sens de la présente invention, lorsque le terme « niveau » est employé en lien avec le positionnement de transducteur(s) ultrasonique(s) distincts au sein de la cuve de macération, il doit donc être logiquement compris comme désignant la hauteur à laquelle est/sont positionné(s) ledit/lesdits transducteur(s) ultrasonique(s) distinct(s) du « niveau » concerné, au sein de la cuve de macération, par rapport au plan horizontal de référence, à savoir le plan horizontal contenant le point le plus bas de ladite cuve de macération.

### Brève description des dessins

Certains aspects de l'invention seront mieux appréhendés à la lecture de la description présentée ci-après, faite en partie en référence aux figures 1 et 2a - 2e, dans lesquelles :
- la figure 1 est un schéma du dispositif selon un mode de réalisation préféré de l'invention, et
- les figures 2a à 2e sont des schémas illustrant le fonctionnement du pressoir à plateau incliné (également dénommé « presse à plateau incliné ») correspondant à la référence numérique 2 sur la figure 1.

### Description détaillée

La description détaillée ci-après a pour but d'exposer l'invention de manière suffisamment claire et complète, notamment à l'aide d'exemples, mais ne doit en aucun cas être regardée comme limitant l'étendue de la protection aux modes de réalisation particuliers et aux exemples présentés ci-après.

### Dispositif d'obtention d'un macérât végétal aqueux liquide selon un mode de réalisation de l'invention

Le dispositif d'obtention d'un macérât végétal aqueux liquide selon un mode de réalisation de l'invention comprend au moins une cuve de macération, de préférence un ensemble de deux cuves de macération identiques. Les cuves de macération, adaptées à la mise en œuvre d'une macération végétale assistée par ultrasons (technique permettant notamment d'intensifier le rendement d'extraction tout en diminuant la durée du procédé d'obtention d'un macérât végétal aqueux liquide selon l'invention et en éliminant les déchets), comprennent chacune en leur sein, avantageusement sur leur paroi interne, au moins deux niveaux de transducteur(s) ultrasonique(s) distincts, chaque niveau de transducteur(s) ultrasonique(s) comprenant au moins un, et de préférence au moins deux, transducteurs ultrasoniques ;
ladite au moins une cuve de macération comprenant en outre au moins un moyen d'agitation mécanique.

Le dispositif comprend au moins un organe de pressage (pressoir) et également au moins une centrifugeuse, avantageusement une centrifugeuse à assiettes (centrifugeuse à grande vitesse).

Le dispositif comprend également une pompe de vidange reliée à chacune des deux cuves, adaptée pour conduire, en fin de macération, le macérât végétal aqueux dans un organe de pressage pour obtenir un macérât végétal aqueux liquide (sous forme de suspension aqueuse) et un gâteau de filtrat de plante(s). Ce mode de réalisation « à deux cuves » s'avère particulièrement avantageux. En effet, pendant qu'une cuve est chargée en eau et en plantes par l'utilisateur, l'autre cuve est en train d'opérer l'étape de macération puis de vidange (afin d'obtenir le macérât végétal aqueux liquide selon l'invention) et vice versa. Ce fonctionnement « en alternance » permet d'obtenir un gain de productivité. La suspension aqueuse obtenue après pressage est ensuite conduite vers ladite au moins une centrifugeuse. Après centrifugation, une solution aqueuse est obtenue, au sein de laquelle les composés bioactifs (et de préférence les composés bioactifs hydrophiles) initialement contenus dans le substrat végétal de départ sont dissouts de manière homogène. Cette solution aqueuse (consommable) peut, selon un mode de réalisation préféré, être directement conditionnée dans un contenant adapté, par exemple en ampoules (telles que des ampoules buvables traditionnelles « à deux pointes », des UNICADOSE^{®} etc.). Selon un mode de réalisation particulier, un dispositif de réduction de taille du substrat végétal et/ou un dispositif de séchage (par exemple les deux) sont en outre associés ou connectés au dispositif selon l'invention. Ce dispositif de réduction de taille du substrat végétal et/ou ce dispositif de séchage est/sont adapté(s) pour effectuer au moins une étape de réduction de taille du substrat végétal et/ou au moins une étape de séchage du substrat végétal, par exemple les deux, avant l'étape b),

De préférence, le dispositif comprend en outre un turbidimètre.

De manière préférée, au moins deux transducteurs ultrasoniques sont positionnés sur la paroi interne de la cuve de macération, à au moins deux niveaux différents (dénommés « niveaux de transducteur(s) ultrasonique(s) »).

De préférence, au moins trois transducteurs ultrasoniques sont positionnés sur la paroi interne de la cuve de macération, à trois niveaux différents (« niveaux de transducteur(s) ultrasonique(s) »). Avantageusement, le premier niveau est situé en bas de cuve de macération, le deuxième en milieu de cuve et le troisième en haut de cuve. De préférence, les trois niveaux susvisés sont positionnés sensiblement à équidistance les uns des autres.

Selon un mode de réalisation préféré, au moins deux transducteurs ultrasoniques sont positionnés sur chacun des deux ou trois niveaux susvisés (préférablement sur chacun des trois niveaux susvisés). Avantageusement, entre 8 et 16, de préférence entre 10 et 14, transducteurs ultrasoniques sont répartis sur les trois niveaux de transducteur(s) ultrasonique(s) susvisés. De préférence, 12 transducteurs ultrasoniques sont répartis sur les trois niveaux susvisés.

Le phénomène de transfert hydrodynamique, de chaleur et de matière pour l'extraction solide-liquide est intensifié par les ultrasons. Le positionnement des transducteurs ultrasoniques est défini afin d'obtenir la plus grande efficacité en combinant l'agitation et les ultrasons mais aussi en tenant compte de la forme et du diamètre de la cuve. Les transducteurs ultrasoniques des trois niveaux peuvent être commandés indépendamment suivant le volume de substrat végétal. Chaque transducteur ultrasonique a, de préférence, une puissance comprise entre environ 300 Watts et environ 450 Watts (par exemple entre 300 et 450 Watts).

Selon un mode de réalisation préféré, l'étape de macération assistée par ultrasons est mise en œuvre, au sein de la cuve de macération, à une densité de puissance comprise 1 et 100 Watts par litre, de préférence comprise entre 1 et 50 Watts par litre, préférablement comprise entre 5 et 25 Watts par litre, de manière préférée comprise entre 10 et 20 Watts par litre.

Typiquement, les transducteurs ultrasoniques sont positionnés de façon à avoir des actions mécaniques et physiques directement sur le substrat végétal, notamment lors de l'explosion des bulles de cavitation dans l'eau. La production de micro-jets dirigés vers une surface solide lors de l'implosion des bulles de cavitation permet de libérer les composés bioactifs par détexturation du substrat végétal. Ces micro-jets, par leur détexturation, augmentent la diffusion de l'eau dans le substrat végétal.

Ledit au moins un moyen d'agitation mécanique permet de maintenir le substrat végétal au plus près des transducteurs ultrasoniques pour que la diffusion des ondes ultrasonores sur le substrat végétal se produise de manière efficace. Ledit au moins un moyen d'agitation mécanique peut maintenir une agitation optimale permettant la préparation d'une grande variété de formulations à base de plante(s). De préférence, il s'agit d'au moins un moyen d'agitation à pales (par exemple comprenant une ou plusieurs hélices) ; la forme desdites pales étant adaptée pour permettre un mouvement descendant ou ascendant des plantes et pour faciliter le mouillage du substrat végétal lors de l'incorporation de celui-ci dans l'eau. La vitesse et le sens d'agitation sont variables, ce qui permet de les ajuster en fonction des formulations. Cette modularité spécifique au dispositif selon l'invention permet de traiter les plantes seules ou en mélange, et aussi de traiter toutes les parties de plantes que ce soit le bois, les tiges, les pétales, les feuilles, les parties aériennes, les racines, les rhizomes, les écorces, les fleurs, les graines, les fruits. Selon un mode de réalisation préféré, ledit moyen d'agitation mécanique est un moyen d'agitation à pales équipé de quatre niveaux d'hélice, la plus basse étant la plus proche possible de la paroi de fond de cuve (ladite paroi de fond de cuve étant, de préférence, de forme sensiblement conique, voire conique).

La pompe de vidange est dimensionnée de façon à transférer le produit des cuves de macération, à savoir le macérât végétal aqueux, audit au moins un organe de pressage (pressoir) tout en gardant une homogénéité du mélange. La vitesse de transfert est ajustable en fonction des caractéristiques du substrat végétal (contenant les composés bioactifs, et en particulier les composés bioactifs hydrophiles).

Selon un mode de réalisation préféré, ledit au moins un organe de pressage est un ensemble de pressoirs automatiques « à plateau carré » et présente une surface de préférence environ égale à 0,7 m². La rotation du gâteau de filtrat de plante(s) entre chaque cycle s'effectue par gravité lors du recul du plateau mobile (comme expliqué ci-dessous). Chaque pressoir comprend au moins une grille de filtration. Les grilles de filtration de chaque pressoir ont, de préférence, une maille de 50 à 150 µm.

La pression uniforme exercée sur toute la surface du plateau est, de préférence, comprise entre environ 2 bars et environ 5 bars (par exemple entre 2 et 5 bars). De manière préférée, la pression exercée est égale à environ 3 bars (typiquement de 3 bars). De préférence, le pressage comprend - ou consiste en - deux cycles successifs. La pression est progressive et chaque segment des cycles de pressage peut être ajusté en durée de maintien de la pression pour une pression déterminée.

Lors du fonctionnement des pressoirs, le chargement du macérât végétal aqueux s'effectue par le dessus du pressoir (de préférence de manière automatisée). Le macérât végétal aqueux est alors situé entre un plateau mobile (vertical ou, de préférence, incliné par rapport à la verticale) d'un côté et une paroi fixe (verticale ou, de préférence, inclinée par rapport à la verticale), de l'autre côté. Lors du premier cycle, le pressage s'effectue en avançant le plateau mobile vers ladite paroi fixe. Cette opération permet de réaliser la séparation entre le substrat végétal épuisé, aussi appelé gâteau de filtrat de plante(s), et le macérât végétal aqueux liquide (sous forme de suspension aqueuse). Le fait d'avoir une paroi fixe inclinée par rapport à la verticale s'avère particulièrement avantageux, dans la mesure où, grâce à ladite paroi inclinée, le centre de gravité du gâteau de filtrat de plante(s) est décalé par rapport à sa base et la bascule du gâteau de filtrat de plante(s) se fait automatiquement par un simple recul du plateau mobile. A l'issue de la bascule, le gâteau de filtrat de plante(s) s'effondre sur lui-même. Ainsi, lors du deuxième (ou second) cycle de pressage, la pression sur le gâteau de filtrat de plante(s) s'exerce selon un axe différent de celui utilisé lors du premier cycle, ce qui permet d'augmenter l'efficacité de l'étape de pressage. Le déchargement du gâteau de filtrat de plante(s) ainsi pressé est effectué par ouverture du pressoir par le dessous, de préférence de manière automatisée.

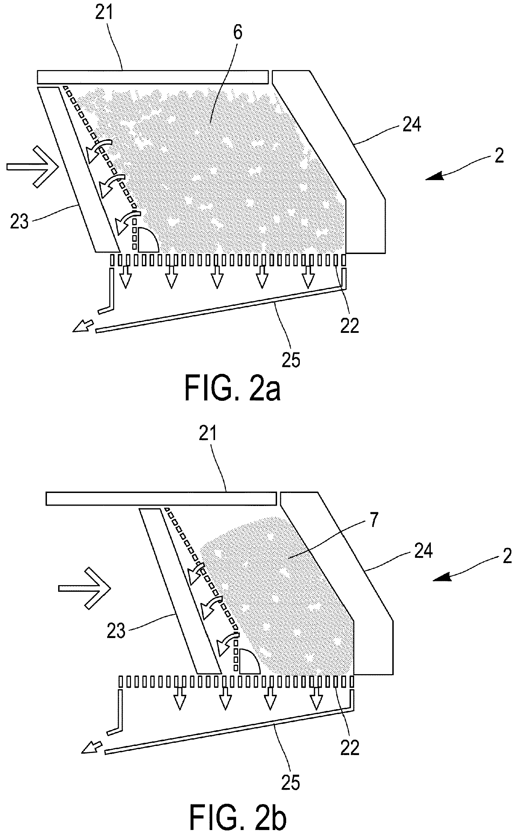

Selon un mode de réalisation particulièrement préféré, l'organe de pressage est un pressoir à plateau incliné 2 (abrégé ci-après en « pressoir 2 », à des fins de concision), dont le fonctionnement est décrit ci-après en références aux figures 2a - 2e. Comme représenté sur ces figures, ce pressoir 2 comprend :
- un couvercle coulissant 21 (adapté pour permettre l'alimentation - « par le haut »
- du pressoir 2 en macérât végétal aqueux 6)
- au moins une grille de filtration 22 (ou un ensemble de grilles de filtration 22, adaptée(s) pour permettre la filtration du macérât végétal aqueux liquide, sous forme de suspension aqueuse),
- un plateau mobile incliné 23,
- une paroi fixe inclinée (ou plateau fixe incliné) 24, et
- une zone de récupération du macérât végétal aqueux liquide 25.

En fin de macération, le produit des cuves de macération (ou de la cuve de macération), à savoir le macérât végétal aqueux 6, est transféré au pressoir 2 sous l'effet de la pompe de vidange (tel qu'expliqué précédemment), de préférence de manière automatisée. Le couvercle coulissant du pressoir 2 coulisse (de préférence de manière automatisée) afin de créer une ouverture par laquelle ledit macérât végétal aqueux 6 pénètre au sein du pressoir 2 (non représenté).

Le couvercle coulissant du pressoir 2 coulisse alors de nouveau, dans le sens opposé, pour refermer ledit pressoir 2 contenant le macérât végétal aqueux 6 avant que le premier cycle de pressage (première opération de pressage) ne commence (non représenté).

Lors du premier cycle de pressage (cf. figures 2a et 2b), le plateau mobile incliné 23 avance en direction de la paroi fixe inclinée 24, compressant le macérât végétal aqueux 6 emprisonné entre, d'une part, le plateau mobile incliné 23 et, de l'autre, la paroi fixe inclinée 24, jusqu'à obtention :
- d'un macérât végétal aqueux liquide, lequel passe à travers ladite au moins une grille de filtration 22 pour arriver au niveau de la zone de récupération du macérât végétal aqueux liquide 25 ; et
- d'un gâteau de filtrat de plante(s) 7.

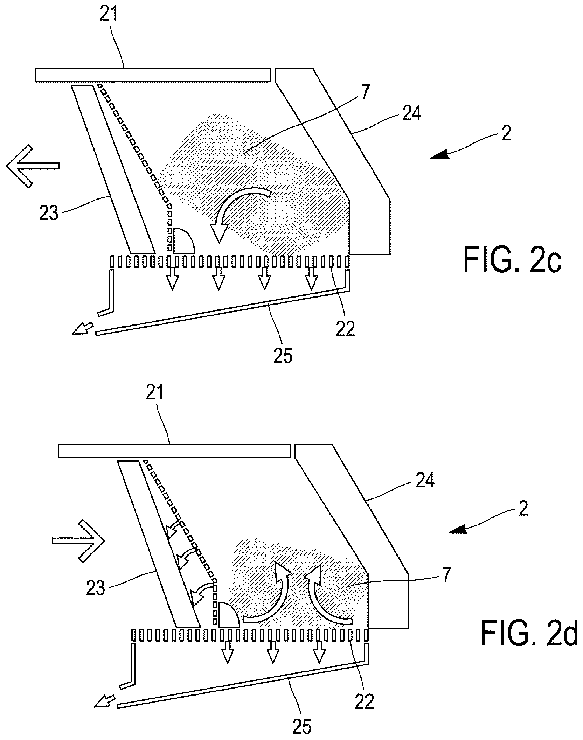

A l'issue de ce premier cycle de pressage, le plateau mobile incliné 23 recule en direction de sa position initiale et le gâteau de filtrat de plante(s) 7 - n'étant plus compressé entre le plateau mobile incliné 23 et la paroi fixe inclinée 24 - bascule (comme représenté en figure 2c), son centre de gravité étant décalé par rapport à sa base.

S'ensuit alors un second cycle de pressage (seconde opération de pressage), au cours duquel le gâteau de filtrat de plante(s) 7, effondré sur lui-même, se trouve compressé entre le plateau mobile incliné 23 et la paroi fixe inclinée 24, le premier avançant en direction de la seconde (cf. figure 2d) comme durant le premier cycle de pressage.

Ce second cycle de pressage, lors duquel la pression est exercée sur le gâteau de filtrat de plante(s) suivant un axe différent de celui suivant lequel la pression est exercée lors du premier cycle de pressage, permet de recueillir, au niveau de la zone de récupération du macérât végétal aqueux liquide 25, davantage de macérât végétal aqueux liquide et, ce faisant, d'augmenter le rendement d'extraction.

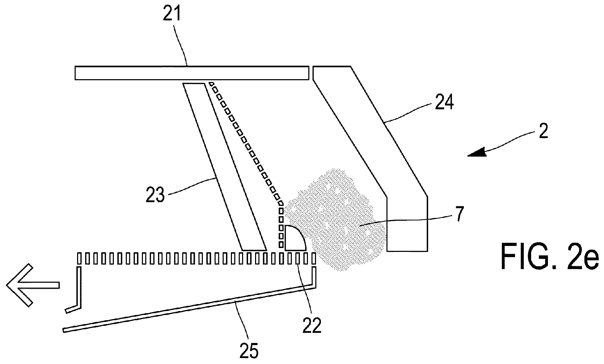

Comme représenté en figure 2e, en fin de second cycle de pressage, la partie inférieure du pressoir 2, comprenant ladite au moins une grille de filtration 22 et la zone de récupération du macérât végétal aqueux liquide 25 coulisse (de préférence de manière automatisée) afin de permettre l'ouverture du pressoir 2 « par le bas » et le déchargement du gâteau de filtrat de plante(s) 7 ainsi pressé. Postérieurement au déchargement du gâteau de filtrat de plante(s) 7 ainsi pressé, le plateau mobile incliné 23 retourne en position initiale, et le pressoir 2 est de nouveau prêt à recevoir le macérât végétal aqueux 6 en provenance des cuves de macération (ou de la cuve de macération), tel qu'expliqué précédemment.

La centrifugeuse du dispositif selon l'invention est, de préférence, une centrifugeuse à assiettes (centrifugeuse à grande vitesse). L'éjection des particules troubles, c'est-à-dire la décharge, s'effectue au niveau de l'espace entre les assiettes de la centrifugeuse en fonctionnement à plein régime. L'alimentation du macérât végétal aqueux liquide (sous forme de suspension aqueuse) se fait par le bas de la centrifugeuse afin de minimiser le volume mort et la présence d'air à l'intérieur du bol, ceci étant avantageux pour des produits sensibles à l'oxydation. La vitesse est comprise entre 3000 et 10000 tours par minute (rpm). De manière préférée, la vitesse est de 6200 tours par minute de sorte que la force exercée en périphérie du bol est de 12000 g. Le débit est compris entre 5 et 15 m³ par heure. De manière préférée, le débit en cours de procédé est de 10 m³ par heure.

De manière optimale, le turbidimètre est positionné en sortie de la centrifugeuse ce qui permet de régler les décharges soit en temps, soit en fonction de la turbidité. Le contrôle de la turbidité s'appuie sur le principe de l'absorption de la lumière dans le liquide. Le turbidimètre s'étalonne automatiquement après chaque vidange dans la pompe de vidange. La lecture après la première vidange en début de production est utilisée comme référence. Un intervalle de temps est défini entre deux vidanges. Un point de déclenchement de la vidange est paramétré à 50% de la plage de réglage de la turbidité. La valeur de ce point de déclenchement est définie en fonction de la turbidité du produit.

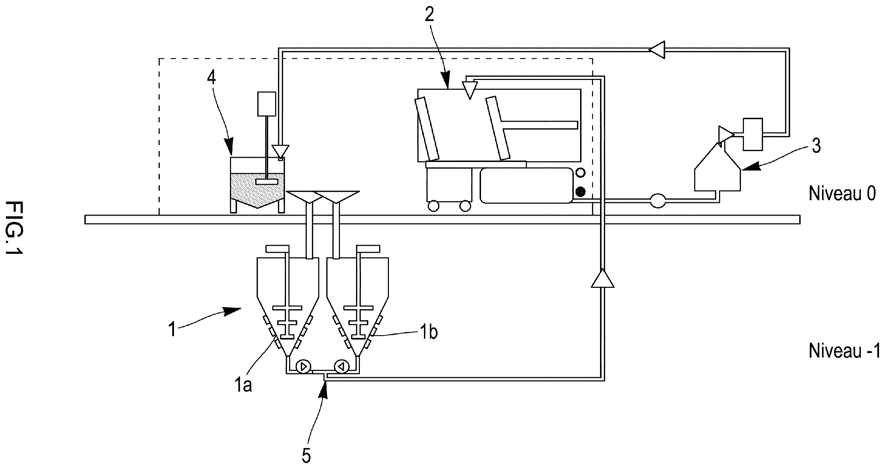

A titre illustratif, un dispositif (ou installation) selon un mode de réalisation préféré de l'invention est schématisé en figure 1. Ce dispositif, automatisé et permettant un fonctionnement en flux continu (à savoir sans interruption ni intervention humaine ; une simple supervision par un opérateur pouvant, le cas échéant, être réalisée), est parfaitement adapté à la mise en œuvre du procédé d'obtention d'un macérât végétal aqueux liquide selon l'invention. Comme représenté en figure 1, ce dispositif comprend les éléments suivants :
- un ensemble 1 de deux cuves de macération identiques 1a, 1b (ayant une partie supérieure cylindrique et une partie inférieure conique), fonctionnant «en alternance », tel qu'expliqué précédemment, chacune des deux cuves de macération comprenant, sur sa paroi interne, trois « niveaux de transducteur(s) ultrasonique(s) », tel que défini précédemment, le premier niveau étant situé en bas de cuve de macération, le deuxième en milieu de cuve et le troisième en haut de cuve (le premier niveau comprenant deux transducteurs ultrasoniques, le deuxième niveau comprenant quatre transducteurs ultrasoniques et le niveau du haut comprenant six transducteurs ultrasoniques), et au moins un moyen d'agitation mécanique (en l'espèce un moyen d'agitation à pales), l'association de l'agencement spécifique des transducteurs ultrasoniques et du moyen d'agitation mécanique permettant d'obtenir une efficacité optimale en termes de détexturation du substrat végétal due au phénomène de cavitation induit par le traitement par ultrasons ;
- un pressoir à plateau incliné 2 (également dénommé « presse à plateau incliné ») dont la fonction est décrite supra (et dont le fonctionnement est schématisé au sein des figures 2a - 2e) ;
- une centrifugeuse à assiettes 3 (centrifugeuse à grande vitesse), dont la fonction est également décrite précédemment ;
- un turbidimètre positionné en sortie de la centrifugeuse à assiettes (non représenté car est intégré dans le conduit de la centrifugeuse 3), permettant de régler les décharges soit en temps, soit en fonction de la turbidité, tel qu'expliqué précédemment ; et
- une cuve de récupération 4, permettant de récupérer le macérât végétal aqueux liquide purifié en sortie de centrifugeuse.

Le dispositif schématisé en figure 1 comprend également une pompe de vidange 5 reliée à chacune des deux cuves de macération 1a, 1b, adaptée pour conduire/transférer, en fin de macération, le macérât végétal aqueux dans le pressoir à plateau incliné 2.

Comme représenté en figure 1, les différents éléments du dispositif énumérés ci-dessus sont connectés les uns aux autres par un système de conduites (correctement dimensionnées) (en d'autres termes ces éléments peuvent être regardés comme étant en communication de fluide). Ceci permet d'éviter les manipulations et/ou interruptions entre lesdits éléments et, par conséquent, de réduire le temps homme/jour tout en gagnant en efficacité, en robustesse et en reproductibilité.

### Procédé selon un mode de réalisation de l'invention

Le procédé selon un mode de réalisation de l'invention, mettant en œuvre le dispositif décrit ci-dessus, est décrit ci-après.

Les différentes étapes du procédé de production du macérât végétal aqueux liquide utilisant l'eau comme solvant à partir d'un ou plusieurs substrat(s) végétal(aux) sont effectuées dans un certain ordre, avec des paramètres adaptés pour une meilleure diffusion et disponibilité des métabolites (composés bioactifs) allant jusqu'à doubler le rendement d'extraction.

La première étape - optionnelle - est une étape de prétraitement du substrat végétal qui peut être frais ou sec. Il peut subir un séchage naturel ou forcé et/ou une réduction de taille. Cette action/ces actions a/ont pour but d'augmenter la disponibilité à l'extraction par macération assistée par ultrasons des structures végétales contenant les composés bioactifs d'intérêt, qui peuvent être notamment sous forme de trichomes ou glandes, internes ou externes vis à vis de la structure du substrat végétal.

La deuxième étape est une étape de macération assistée par ultrasons ( mettant en jeu une pluralité de transducteurs ultrasoniques pouvant de préférence travailler en simultané ou en alternance, avec le susdit au moins un moyen d'agitation mécanique afin d'opérer une extraction solide-liquide et obtenir ainsi une suspension liquide). Cette étape permet de moduler l'extraction par macération en fonction du substrat végétal ou du mélange de végétaux ainsi que des composés bioactifs d'intérêt présents. Le substrat végétal utilisé lors de l'étape de macération peut être seul ou en mélange et peut consister, par exemple, en des fruits, fleurs et/ou feuilles.

La troisième étape est une étape de séparation du macérât végétal aqueux liquide et du solide végétal épuisé (gâteau de filtrat de plante(s)), réalisée à partir du macérât végétal aqueux obtenu par macération aqueuse. Cette étape de séparation est effectuée par l'ensemble de pressoirs automatiques susvisé permettant, d'une part, la séparation du macérât végétal aqueux liquide et, d'autre part, d'exsuder le macérât végétal aqueux liquide restant emprisonné dans les fibres végétales par une succession de cycles de pressage qui agit comme un pompage.

La quatrième étape comprend une centrifugation du macérât végétal aqueux liquide (sous forme de suspension aqueuse) pour éliminer les suspensions encore potentiellement présentes et préparer une solution buvable, avantageusement sous forme de solution aqueuse, adaptée pour une mise en formulation directe et/ou une mise en ampoules. Cette étape de centrifugation est particulièrement avantageuse notamment en ce qu'elle évite une étape de filtration sur une plaque et permet d'obtenir un liquide clarifié limpide permettant directement un conditionnement en ampoules (et ne présentant pas de traces de solvant(s) organique(s)).

Le pourcentage massique de substrat végétal utilisé lors de l'étape de macération assistée par ultrasons est avantageusement compris entre 1 % et 20%, de préférence entre 5% et 15% (par rapport à la masse totale substrat végétal + eau).

Le solvant utilisé lors de l'étape de macération est uniquement de l'eau qui peut être sous forme naturelle ou déminéralisée.

De façon avantageuse, et contrairement aux procédés de l'état de la technique mettant en œuvre des solvants organiques, le procédé selon l'invention permet de produire un macérât végétal aqueux liquide riche en composés bioactifs (de préférence hydrophiles) représentatifs de la grande variété de composés bioactifs du ou des végétaux extraits par ledit procédé, le tout sans solvant organique résiduel (même à l'état de traces).

En outre, de manière avantageuse, et contrairement aux procédés de l'état de la technique mettant en œuvre de l'eau par décoction ou macération, le procédé de l'invention permet de produire un macérât végétal aqueux liquide plus riche en composés bioactifs (de préférence hydrophiles). Le rendement issu de ce procédé peut être supérieur de 10% à 100% par rapport aux procédés de l'état de la technique mettant en œuvre de l'eau par décoction ou macération.

Typiquement, les composés bioactifs (de préférence hydrophiles) extraits dans le macérât végétal aqueux liquide incluent des composés issus du métabolisme primaire des plantes glucides, lipides, aminoacides, peptides et protéines, mais aussi des composés issus du métabolisme secondaire : notamment des composés phénoliques, des composés de type terpénoides, des composés de type alcaloïde.

Typiquement, le substrat végétal peut être choisi parmi divers organes, tissus et structures de la plante, assemblés ou séparés (par exemple : écorce, tiges (rhizome...), fleurs (pétales, sépales, feuilles, racines, les graines, les fruits ... ). Il peut être utilisé seul ou en association avec d'autres végétaux. Le matériel végétal peut être choisi parmi la liste non-exhaustive suivante *: Harpagophytum procumbens, Harpagophytum zeyheri, Ribes nigrum, Salix alba, Urtica dioica, Urtica urens, Crataegus monogyna, Crataegus laevigata, Crataegus.nigra, Crataegus.pentagyna, Crataegus azarolus, Passiflora incarnata, Citrus aurantium, Lavandula angustifolia, Arctostaphylos uva-ursi, Betula pendula, Betula pubescens, Calluna vulgaris, Hamamelis virginiana, Vitis vinifera, Ruscus aculeatus, Rosmarinus officinalis, Cynara scolymus, Angelica archangelica, Olea europea.*

Le procédé selon l'invention permet de produire un macérât végétal aqueux liquide riche en composés bioactifs (de préférence hydrophiles) à partir de substrats végétaux, le résidu solide étant donc appauvri.

Le procédé selon l'invention permet également de produire un résidu solide, à savoir un gâteau de filtrat de plante(s), qui ne contient aucun solvant organique ou contaminant, et peut être directement composté. Ceci représente un avantage significatif.

Selon un mode de réalisation, le procédé selon l'invention peut comprendre en outre une étape supplémentaire de formulation/conditionnement du macérât végétal aqueux liquide.

Le procédé selon l'invention permet en outre de produire un macérât végétal aqueux liquide issu d'un substrat végétal comprenant des composés bioactifs et de l'eau, caractérisé en ce que la concentration massique avec ledit procédé sous ultrasons permet une augmentation significative de l'ordre de 25% à 250% du rendement global, de la matière sèche et/ou de la teneur en composés bioactifs par rapport à un procédé conventionnel d'infusion.

Contre toute attente, le procédé selon l'invention permet également de produire un macérât végétal aqueux liquide significativement plus riche en ADN par rapport aux procédés de l'art antérieur. En effet, la quantité d'ADN extraite des cellules végétales et retrouvée dans le macérât végétal aqueux liquide selon l'invention (79 microgrammes d'ADN par gramme d'extrait de romarin), est supérieure de 100% par rapport au procédé conventionnel par infusion (33,9 microgrammes d'ADN par gramme d'extrait de romarin).

Le procédé selon l'invention permet en outre de produire un extrait (macérât végétal aqueux liquide), avec un rendement au minimum équivalent au procédé d'infusion conventionnel, directement formulable en une durée d'extraction réduite par un facteur de 2 à 3.

Selon un mode de réalisation préféré, le procédé selon l'invention fonctionne de manière semi-continue. En effet, et tel qu'indiqué précédemment, les deux cuves fonctionnent de manière décalée (en alternance) l'une par rapport à l'autre. Ainsi, pendant qu'une cuve est chargée en eau et en plantes par l'utilisateur, l'autre cuve est en train d'opérer l'étape de macération puis de vidange. Par ailleurs, le dispositif selon l'invention est avantageusement entièrement automatisé.

Le procédé selon l'invention et le dispositif selon l'invention permettent d'augmenter la productivité et de réduire le temps homme/jour par rapport aux procédés et dispositifs conventionnels, et permettent aussi une performance industrielle de l'ensemble des étapes du procédé.

Ainsi, le procédé et le dispositif selon l'invention répondent aux besoins de produire des macérâts végétaux aqueux liquides comprenant une grande variété de composés bioactifs (de préférence hydrophiles), sans solvant organique ni transformation chimique. Le procédé est modulable pour s'adapter à une large variété de plantes aromatiques et médicinales sous forme de fleurs, fruits ou feuilles, seul ou en mélange. Le procédé intègre plusieurs opérations unitaires avec, la préparation du végétal par séchage et/ou réduction de taille du substrat végétal (opération optionnelle), la macération par extraction solide-liquide assistée par ultrasons dans l'eau comme solvant, la séparation des phases solide-liquide par un pressoir, et finalement l'obtention dudit macérât végétal aqueux liquide parfaitement clair grâce à une centrifugeuse (mode de réalisation particulièrement préféré de l'invention).

Selon un mode de réalisation, et notamment grâce à un agencement spécifique des transducteurs ultrasoniques, le procédé selon l'invention permet l'extraction solide-liquide du substrat végétal avec un solvant contenant de l'eau pour obtenir d'une part une fraction liquide consistant en le macérât végétal aqueux liquide avec une grande variété de composés bioactifs représenté en particulier par les composés hydrophiles dudit substrat végétal et d'autre part un résidu solide épuisé et sans trace de solvant organique qui pourra être composté.

### Exemples

Dans les exemples ci-après, la méthode pour déterminer la teneur en matière sèche est la suivante : dépôt de 2g de macérât végétal aqueux liquide pesé avec précision (m1(g)) sur une balance dans un cristallisoir en verre, passage à l'étuve à 105 °C pendant 3 h du cristallisoir contenant le macérât végétal aqueux liquide, pesée du résidu sec obtenu (m2(g)), expression du taux résultant de matière sèche en pourcentage : [m2(g) / m1(g)] x100.

Cette mesure permet de calculer les quantités d'extrait sec théorique obtenu lors de l'opération d'extraction avec la formule suivante : (masse de macérât végétal aqueux liquide) x (taux de matière sèche) = Quantité de matière sèche extraite.

Afin de mesurer la performance globale d'extraction, le calcul du rendement d'extraction est donné en % (m/m) par la formule suivante : [(Quantité d'extrait sec théorique (Kg)) / (Quantité de plante mise en œuvre (Kg))] x100

Egalement des dosages précis par Chromatographie Liquide Haute Performance (CLHP) de métabolites secondaires d'intérêt (composés bioactifs) sont donnés, afin de compléter la mesure de performance du procédé d'extraction. Les résultats sont exprimés en mg de composé(s) bioactif(s)/ml de macérât végétal aqueux liquide. Le détail est donné dans chaque exemple avec des conditions spécifiques aux plantes ou mélanges de plantes utilisés.

### Exemple 1

Le premier exemple concerne l'extraction de racines d'Harpagophytum.

La teneur en harpagosides est obtenue par CLHP dans les conditions suivantes : Colonne Hypersil C18 150 x 4,6 mm - 5 µm, température : 25°C, mode isocratique phase mobile : Méthanol R / Eau purifiée (50/50) (V/V), débit 1,5 ml/min, Détection : spectrophotomètre UV/Visible à 278 nm, temps d'analyse : 15 min.

Le dosage est réalisé par étalonnage externe avec témoin Harpagoside.

Une masse de 123,435 kg de racines d'Harpagophytum, sous forme sèche et ayant préalablement subi une réduction de taille, est mise en contact avec 1234,350 kg d'eau dans les cuves comprenant les transducteurs ultrasoniques d'une puissance de 3600 Watt et n'excédant pas 5400 W pour une durée de 45 minutes. On obtient alors, après les étapes de pressage avec une pression d'environ 3 bars et de centrifugation (12000 g environ), une quantité de 918 kg de macérât végétal aqueux liquide contenant une grande variété de composés bioactifs (en particulier hydrophiles) de la racine. Les analyses montrent que le taux de matière sèche est de 5,982% et la teneur en harpagosides est de 0,8747 mg/ml, ce qui correspond à un rendement de 44,49%. Le traitement des racines d'harpagophytum par un procédé conventionnel d'infusion utilisé en industrie qui dure au minimum 120 minutes, donne un rendement de 30,61 % avec une matière sèche de 2,666% et la teneur en harpagosides est de 0,2787 mg/ml. Le procédé selon l'invention permet donc une diminution du temps de procédé (temps divisé par 2,66) et également une augmentation du rendement (x 1,45), une augmentation de la matière sèche (x 2,24) et une augmentation de la teneur en harpagosides (x 3,13).

### Exemple 2

Le deuxième exemple concerne l'extraction d'un mélange de feuilles d'Hamamelis (38,7% m/m), de racines de Fragon (38.7% m/m) et de feuilles de Vigne rouge (22,6 % m/m).

La teneur en isoquercitroside est obtenue par CLHP dans les conditions suivantes : Colonne Synergi Hydro RP C18 100 x 3 mm - 2,5 µm, température : 40°C, mode gradient phase mobile : Eau/Acétonitrile, débit 0,3 ml/min, Détection : spectrophotomètre UV/Visible à 350 nm, temps d'analyse : 20 min.

Le dosage est effectué par étalonnage externe avec témoin Isoquercitroside.

Une masse de 41,8 kg du mélange de plantes, sous forme sèche et ayant préalablement subi une réduction de taille, est mise en contact avec 952,54 Kg d'eau dans les cuves comprenant les transducteurs ultrasoniques d'une puissance de 3600 Watt et n'excédant pas 5400 W pour une durée de 45 minutes. On obtient alors, après les étapes de pressage avec une pression d'environ 3 bars et de centrifugation (12000g environ), une quantité de 763 kg de macérât végétal aqueux liquide contenant une grande variété de composés bioactifs (en particulier hydrophiles) du mélange de plantes. Les analyses montrent que le taux de matière sèche est de 1.07% et la teneur en isoquercitroside est de 0,1029 mg/ml, ce qui correspond à un rendement de 19.5%. Le traitement du mélange de plantes par un procédé conventionnel d'infusion utilisé en industrie qui dure au minimum 120 minutes, donne un rendement de 11.36% avec une matière sèche de 0,396 % et la teneur en isoquercitroside est de 0,0223mg/ml. Le procédé selon l'invention permet une diminution du temps de procédé (temps divisé par 2,66), et également une augmentation du rendement (x 1,71), une augmentation de la matière sèche (x 2,70) et une augmentation de la teneur en isoquercitroside (x 4,61).

### Exemple 3

Le troisième exemple concerne l'extraction d'un mélange de feuilles d'Artichaut (33,3 % m/m), de feuilles de Romarin (33,3 % m/m) et de racines d'Angélique (33,3 % m/m).

La teneur en acide rosmarinique est obtenue par CLHP dans les conditions suivantes : Colonne Zorbax SB C18 50 mm x 2,1 mm -1,8 µm, température : 30°C, mode gradient phase mobile : Eau / Acétonitrile, débit 0,95 ml/min, Détection : spectrophotomètre UV/Visible à 320 nm, temps d'analyse : 17 min

Le dosage est effectué par étalonnage externe avec témoin d'acide rosmarinique.

Une masse de 90 kg du mélange de plantes, sous forme sèche et ayant préalablement subi une réduction de taille, est mise en contact avec 897,3 Kg d'eau dans les cuves comprenant les transducteurs ultrasoniques d'une puissance de 3600 Watt et n'excédant pas 5400 W pour une durée de 45 minutes. On obtient alors, après les étapes de pressage avec une pression d'environ 3 bars et de centrifugation (12000g environ), une quantité de 640 Kg de macérât végétal aqueux liquide contenant une grande variété de composés bioactifs (en particulier hydrophiles) du mélange de plantes. Les analyses montrent que le taux de matière sèche est de 2,265 % et la teneur en acide rosmarinique est de 0,2664 mg/ml, ce qui correspond à un rendement de 16,1%. Le traitement du mélange de plantes par un procédé conventionnel d'infusion utilisé en industrie qui dure au minimum 120 minutes, donne un rendement de 10,8 % avec un taux de matière sèche de 0,815 % et la teneur en acide rosmarinique de 0,0303mg/ml. Le procédé selon l'invention permet donc une diminution du temps de procédé (temps divisé par 2,66), et également une augmentation du rendement (x 1,49) une augmentation de la matière sèche (x 2,78), et une augmentation de la teneur en acide rosmarinique (x 8,79).

### Références bibliographiques

[1] : SUSLICK K.S., « Ultrasound : chemical and physical and biological effects », Wiley-VCH, London, (1988)
[2] : CHEMAT et al, « Ultrasound assisted extraction of food and natural products. Mechanisms, techniques, combinations, protocols and applications », Ultrasonics Sonochemistry (2017), 34, 540-560.

## Revendications

1. Procédé d'obtention d'un macérât végétal aqueux liquide, ledit procédé comprenant les étapes suivantes :
a) fourniture d'un substrat végétal,
b) macération dudit substrat végétal dans l'eau par macération assistée par ultrasons, jusqu'à obtention d'un macérât végétal aqueux,
ladite macération étant effectuée dans au moins une cuve de macération (1a, 1b) comprenant en son sein, avantageusement sur sa paroi interne, au moins deux niveaux de transducteur(s) ultrasonique(s) distincts, chaque niveau de transducteur(s) ultrasonique(s) comprenant au moins un, et de préférence au moins deux, transducteurs ultrasoniques,
c) obtention d'un macérât végétal aqueux liquide par séparation du macérât végétal aqueux liquide et du substrat végétal, ladite séparation étant réalisée par pressage du macérât végétal aqueux obtenu à l'étape b), de préférence sous une pression uniforme comprise entre environ 2 bars et environ 5 bars, préférablement entre environ 2,5 bars et environ 3,5 bars, avantageusement d'environ 3 bars.

2. Procédé selon la revendication 1, dans lequel l'étape b) est effectuée partiellement ou entièrement, de préférence entièrement, sous agitation.

3. Procédé selon la revendication 1 ou 2, comprenant, après l'étape c), les étapes suivantes :
d) purification, de préférence par centrifugation, du macérât végétal aqueux liquide obtenu à l'étape c) de manière à obtenir un macérât végétal aqueux liquide purifié, avantageusement sous forme de solution aqueuse,
e) récupération du macérât végétal aqueux liquide ainsi purifié.

4. Procédé selon la revendication précédente, ledit procédé comprenant, entre les étapes d) et e), les étapes suivantes :
d1) mesure de la turbidité du macérât végétal aqueux liquide purifié à l'étape d),
d2) comparaison de la turbidité mesurée à l'étape d1) avec un seuil de turbidité prédéterminé, et
- d3) élimination dudit macérât végétal aqueux liquide purifié lorsque la turbidité mesurée à l'étape d1) est supérieure audit seuil de turbidité, ou
- d3') récupération, à l'étape e), du macérât végétal aqueux liquide purifié lorsque la turbidité mesurée à l'étape d1) est inférieure ou égale audit seuil de turbidité.

5. Procédé selon la revendication 3 ou 4, dans lequel l'étape d) est au moins une étape de centrifugation, dont les paramètres comprennent de préférence :
- une vitesse de centrifugation comprise entre 3000 et 10000 rpm, préférablement supérieure à 5000 rpm et inférieure ou égale à 10000 rpm, avantageusement comprise entre 5200 et 7500 rpm, de manière particulièrement préférée comprise entre 6000 et 7000 rpm, et/ou
- un débit en macérât végétal aqueux liquide purifié compris entre 5 et 15 m³/h, de préférence entre 7 et 13 m³/h, préférablement entre 8 et 12 m³/h, avantageusement entre 9 et 11 m³/h, de manière préférée d'environ 10 m³/h,
avantageusement les deux.

6. Procédé selon l'une des revendications précédentes, dans lequel ladite étape c) comprend au moins deux opérations de pressage, de préférence réalisées de manière successive.

7. Procédé selon la revendication précédente, dans lequel lesdites au moins deux opérations de pressage dudit macérât végétal aqueux sont réalisées de manière successive, et dans lequel la pression exercée sur ledit macérât végétal aqueux lors de la deuxième opération de pressage est exercée suivant un axe différent de l'axe suivant lequel la pression est exercée sur le macérât végétal aqueux lors de la première opération de pressage.

8. Procédé selon l'une des revendications précédentes, dans lequel l'étape b) est réalisée à une température de 20°C à 80°C, de préférence de 30°C à 75°C, préférablement de 40°C à 70°C, avantageusement de 55°C à 65°C.

9. Procédé selon l'une des revendications précédentes, comprenant, avant l'étape b), au moins une étape de prétraitement dudit substrat végétal, telle qu'au moins une étape de séchage et/ou de réduction de taille du substrat végétal, avantageusement les deux.

10. Procédé selon l'une des revendications 1 à 9, ladite macération étant effectuée dans au moins une cuve de macération (1a, 1b) comprenant en son sein, avantageusement sur sa paroi interne, au moins trois niveaux de transducteur(s) ultrasonique(s) distincts, chaque niveau de transducteur(s) ultrasonique(s) comprenant au moins un, et de préférence au moins deux, transducteurs ultrasoniques,

11. Procédé de conditionnement d'un macérât végétal aqueux liquide dans un contenant à usage pharmaceutique, cosmétique, nutritionnel ou vétérinaire, tel qu'un flacon, un sachet unidose, un stick unidose, une flaconnette, une ampoule bouteille unidose ou une ampoule, dans lequel ledit macérât végétal aqueux liquide est le macérât végétal aqueux liquide obtenu à l'étape c) ou à l'étape e), lorsque l'étape e) est présente, du procédé selon l'une des revendications précédentes, ledit procédé de conditionnement comprenant l'/les étape(s) suivante(s) :
- le cas échéant procéder à l'ouverture du contenant si celui-ci est obturé,
- remplir, par tout moyen, ledit contenant totalement ou partiellement à l'aide dudit macérât végétal aqueux liquide puis, le cas échéant,
- obturer ledit contenant ainsi rempli, de préférence de manière hermétique.

12. Dispositif d'obtention d'un macérât végétal aqueux liquide, adapté pour mettre en œuvre le procédé selon l'une des revendications 1 à 10, ledit dispositif comprenant :
i) au moins une cuve de macération (1a, 1b) comprenant en son sein, avantageusement sur sa paroi interne, au moins deux niveaux de transducteur(s) ultrasonique(s) distincts, chaque niveau de transducteur(s) ultrasonique(s) comprenant au moins un, et de préférence au moins deux, transducteurs ultrasoniques,
ladite au moins une cuve de macération (1a, 1b) comprenant en outre au moins un moyen d'agitation mécanique adapté pour maintenir le substrat végétal à une distance prédéterminée des transducteurs ultrasoniques, ladite au moins une cuve de macération (1a, 1b) étant adaptée pour recevoir le substrat végétal, l'eau et effectuer l'étape b),
ii) au moins un organe de pressage (2), tel qu'au moins un pressoir (2), adapté pour recevoir, à l'issue de l'étape b), le macérât végétal aqueux et effectuer l'étape c),
iii) de préférence au moins une centrifugeuse (3), préférablement une centrifugeuse à assiettes (3), adaptée pour recevoir le macérât végétal aqueux liquide obtenu à l'étape c) et effectuer l'étape d), lorsque le procédé comprend ladite étape d),
iv) avantageusement au moins un turbidimètre, adapté pour contrôler la turbidité du macérât végétal aqueux liquide, préférablement obtenu à l'étape d), lorsque le procédé comprend ladite étape d).

13. Dispositif selon la revendication 12, dans lequel ladite au moins une cuve de macération (1a, 1b) comprend en son sein, avantageusement sur sa paroi interne, au moins trois niveaux de transducteur(s) ultrasonique(s) distincts ; chaque niveau de transducteur(s) ultrasonique(s) comprenant au moins un, et de préférence au moins deux, transducteurs ultrasoniques.

14. Dispositif selon la revendication 12 ou 13, dans lequel ladite au moins une cuve de macération (1a, 1b), ledit au moins un organe de pressage (2), ladite au moins une centrifugeuse (3) - lorsqu'elle est présente -, et ledit au moins un turbidimètre - lorsqu'il est présent-, sont connectés entre eux par un système de conduites.

15. Dispositif selon l'une des revendications 12 à 14, ledit dispositif étant automatisé.

## Patentansprüche

1. Ein Verfahren zum Erhalten eines flüssigen wässrigen Pflanzenmazerats, wobei das Verfahren die folgenden Schritte beinhaltet:
a) Bereitstellen eines Pflanzensubstrats,
b) Mazerieren des Pflanzensubstrats in Wasser durch ultraschallunterstütztes Mazerieren bis zum Erhalt eines wässrigen Pflanzenmazerats,
wobei das Mazerieren in mindestens einem Mazerationstank (1a, 1b) durchgeführt wird, der in seinem Inneren, vorteilhafterweise an seiner Innenwand, mindestens zwei getrennte Ebenen von (einem) Ultraschallwandler(n) beinhaltet, wobei jede Ebene von (einem) Ultraschallwandler(n) mindestens einen, und vorzugsweise mindestens zwei, Ultraschallwandler beinhaltet,
c) Erhalten eines flüssigen wässrigen Pflanzenmazerats durch Trennen des flüssigen wässrigen Pflanzenmazerats und des Pflanzensubstrats, wobei das Trennen durch Pressen des in Schritt b) erhaltenen wässrigen Pflanzenmazerats erfolgt, vorzugsweise unter einem gleichmäßigen Druck zwischen etwa 2 bar und etwa 5 bar, bevorzugt zwischen etwa 2,5 bar und etwa 3,5 bar, vorteilhafterweise von etwa 3 bar.

2. Verfahren gemäß Anspruch 1, wobei Schritt b) teilweise oder vollständig, vorzugsweise vollständig, unter Rühren durchgeführt wird.

3. Verfahren gemäß Anspruch 1 oder 2, das nach Schritt c) die folgenden Schritte beinhaltet:
d) Reinigen, vorzugsweise durch Zentrifugieren, des in Schritt c) erhaltenen flüssigen wässrigen Pflanzenmazerats, um ein gereinigtes flüssiges wässriges Pflanzenmazerat, vorteilhafterweise in Form einer wässrigen Lösung, zu erhalten,
e) Rückgewinnen des so gereinigten flüssigen wässrigen Pflanzenmazerats.

4. Verfahren gemäß dem vorhergehenden Anspruch, wobei das Verfahren zwischen Schritten d) und e) die folgenden Schritte beinhaltet:
d1) Messen der Trübung des in Schritt d) gereinigten flüssigen wässrigen Pflanzenmazerats,
d2) Vergleichen der in Schritt d1) gemessenen Trübung mit einem vorbestimmten Trübungsschwellenwert und
- d3) Entfernen des gereinigten flüssigen wässrigen Pflanzenmazerats, wenn die in Schritt d1) gemessene Trübung über dem Trübungsschwellenwert liegt, oder
- d3') Rückgewinnen, in Schritt e), des gereinigten flüssigen wässrigen Pflanzenmazerats, wenn die in Schritt d1) gemessene Trübung kleiner als der oder gleich dem Trübungsschwellenwert ist.

5. Verfahren gemäß Anspruch 3 oder 4, wobei Schritt d) mindestens ein Schritt des Zentrifugierens ist, dessen Parameter vorzugsweise Folgendes beinhalten:
- eine Zentrifugationsgeschwindigkeit zwischen 3 000 und 10 000 U/min, bevorzugt über 5 000 U/min und unter oder gleich 10000 U/min, vorteilhafterweise zwischen 5 200 und 7 500 U/min, in besonders bevorzugter Weise zwischen 6 000 und 7 000 U/min, und/oder
- einen Durchfluss an gereinigtem flüssigen wässrigen Pflanzenmazerat zwischen 5 und 15 m³/h, vorzugsweise zwischen 7 und 13 m³/h, bevorzugt zwischen 8 und 12 m³/h, vorteilhafterweise zwischen 9 und 11 m³/h, in bevorzugter Weise von etwa 10 m³/h,
vorteilhafterweise beides.

6. Verfahren gemäß einem der vorhergehenden Ansprüche, wobei Schritt c) mindestens zwei Pressvorgänge beinhaltet, die vorzugsweise nacheinander durchgeführt werden.

7. Verfahren gemäß dem vorhergehenden Anspruch, wobei die mindestens zwei Pressvorgänge des wässrigen Pflanzenmazerats nacheinander erfolgen und wobei der Druck, der während des zweiten Pressvorgangs auf das wässrige Pflanzenmazerat ausgeübt wird, entlang einer Achse ausgeübt wird, die sich von der Achse unterscheidet, entlang derer der Druck während des ersten Pressvorgangs auf das wässrige Pflanzenmazerat ausgeübt wird.

8. Verfahren gemäß einem der vorhergehenden Ansprüche, wobei Schritt b) bei einer Temperatur von 20 °C bis 80 °C, vorzugsweise von 30 °C bis 75 °C, bevorzugt von 40 °C bis 70 °C, vorteilhafterweise von 55 °C bis 65 °C erfolgt.

9. Verfahren gemäß einem der vorhergehenden Ansprüche, das vor Schritt b) mindestens einen Schritt des Vorbehandelns des Pflanzensubstrats, wie etwa mindestens einen Schritt des Trocknens und/oder des Verkleinerns des Pflanzensubstrats, vorteilhafterweise beides, beinhaltet.

10. Verfahren gemäß einem der Ansprüche 1 bis 9, wobei das Mazerieren in mindestens einem Mazerationstank (1a, 1b) durchgeführt wird, der in seinem Inneren, vorteilhafterweise an seiner Innenwand, mindestens drei getrennte Ebenen von (einem) Ultraschallwandler(n) beinhaltet, wobei jede Ebene von (einem) Ultraschallwandler(n) mindestens einen, und vorzugsweise mindestens zwei, Ultraschallwandler beinhaltet.

11. Ein Verfahren zum Verpacken eines flüssigen wässrigen Pflanzenmazerats in einen Behälter für pharmazeutische, kosmetische, Ernährungs- oder veterinärmedizinische Zwecke, wie etwa eine Flasche, einen Unidose-Beutel, einen Unidose-Stick, ein Fläschchen, eine Unidose-Flaschenampulle oder eine Ampulle, wobei das flüssige wässrige Pflanzenmazerat das flüssige wässrige Pflanzenmazerat ist, das in Schritt c) oder in Schritt e), wenn Schritt e) vorhanden ist, des Verfahrens gemäß einem der vorhergehenden Ansprüche erhalten wird, wobei das Verpackungsverfahren den/die folgenden Schritt(e) beinhaltet:
- falls nötig, Öffnen des Behälters, falls dieser verschlossen ist,
- Befüllen des Behälters ganz oder teilweise mit dem flüssigen wässrigen Pflanzenmazerat auf beliebige Weise und dann, falls nötig,
- Verschließen des so befüllten Behälters, vorzugsweise auf luftdichte Weise.

12. Eine Vorrichtung zum Erhalten eines flüssigen wässrigen Pflanzenmazerats, die zum Implementieren des Verfahrens gemäß einem der Ansprüche 1 bis 10 geeignet ist, wobei die Vorrichtung Folgendes beinhaltet:
i) mindestens einen Mazerationstank (1a, 1b), der in seinem Inneren, vorteilhafterweise an seiner Innenwand, mindestens zwei getrennte Ebenen von (einem) Ultraschallwandler(n) beinhaltet, wobei jede Ebene von (einem) Ultraschallwandler(n) mindestens einen, und vorzugsweise mindestens zwei, Ultraschallwandler beinhaltet,
wobei der mindestens eine Mazerationstank (1a, 1b) ferner mindestens ein mechanisches Rührmittel beinhaltet, das dazu geeignet ist, das Pflanzensubstrat in einem vorbestimmten Abstand von den Ultraschallwandlern zu halten, wobei der mindestens eine Mazerationstank (1a, 1b) dazu geeignet ist, das Pflanzensubstrat und Wasser aufzunehmen und Schritt b) durchzuführen,
ii) mindestens ein Presselement (2), wie etwa mindestens eine Presse (2), die dazu geeignet ist, nach Abschluss von Schritt b) das wässrige Pflanzenmazerat aufzunehmen und Schritt c) durchzuführen,
iii) vorzugsweise mindestens eine Zentrifuge (3), bevorzugt eine Tellerzentrifuge (3), die dazu geeignet ist, das in Schritt c) erhaltene flüssige wässrige Pflanzenmazerat aufzunehmen und Schritt d) durchzuführen, wenn das Verfahren Schritt d) beinhaltet,
iv) vorteilhafterweise mindestens ein Turbidimeter, das dazu geeignet ist, die Trübung des flüssigen wässrigen Pflanzenmazerats, das bevorzugt in Schritt d) erhalten wird, zu überwachen, wenn das Verfahren Schritt d) beinhaltet.

13. Vorrichtung gemäß Anspruch 12, wobei der mindestens eine Mazerationstank (1a, 1b) in seinem Inneren, vorteilhafterweise an seiner Innenwand, mindestens drei getrennte Ebenen von (einem) Ultraschallwandler(n) beinhaltet; wobei jede Ebene von (einem) Ultraschallwandler(n) mindestens einen, und vorzugsweise mindestens zwei, Ultraschallwandler beinhaltet.

14. Vorrichtung gemäß Anspruch 12 oder 13, wobei der mindestens eine Mazerationstank (1a, 1b), das mindestens eine Presselement (2), die mindestens eine Zentrifuge (3) - falls vorhanden - und das mindestens eine Turbidimeter - falls vorhanden - durch ein Leitungssystem miteinander verbunden sind.

15. Vorrichtung gemäß einem der Ansprüche 12 bis 14, wobei die Vorrichtung automatisiert ist.

## Claims

1. A method of obtaining an aqueous liquid plant maceration, said method comprising the following steps:
a) providing a plant substrate,
b) macerating said plant substrate in water by ultrasound-assisted maceration, until an aqueous plant maceration is obtained,
said maceration being performed in at least one maceration tank (1a, 1b) comprising therein, advantageously on its internal wall, at least two distinct levels of ultrasonic transducer(s), each level of ultrasonic transducer(s) comprising at least one, and preferably at least two, ultrasonic transducers,
c) obtaining an aqueous liquid plant maceration by separating the aqueous liquid plant maceration and the plant substrate, said separation being realised by pressing the aqueous plant maceration obtained in step b), preferably under a uniform pressure of between about 2 bar and about 5 bar, preferentially between about 2.5 bar and about 3.5 bar, advantageously of about 3 bar.

2. The method according to claim 1, wherein step b) is performed partially or entirely, preferably entirely, under stirring.

3. The method according to claim 1 or 2, comprising, after step c), the following steps:
d) purifying, preferably by centrifuging, the aqueous liquid plant maceration obtained in step c) so as to obtain a purified aqueous liquid plant maceration, advantageously in the form of aqueous solution,
e) recovering the aqueous liquid plant maceration purified in this manner.

4. The method according to the preceding claim, said method comprising, between steps d) and e), the following steps:
d1) measuring the turbidity of the aqueous liquid plant maceration purified in step d),
d2) comparing the turbidity measured in step d1) to a predetermined turbidity threshold, and
- d3) removing said purified aqueous liquid plant maceration when the turbidity measured in step d1) is above said turbidity threshold, or
- d3') recovering, in step e), the purified aqueous liquid plant maceration when the turbidity measured in step d1) is below or equal to said turbidity threshold.

5. The method according to claim 3 or 4, wherein step d) is at least one centrifugation step, whose parameters comprise preferably:
- a centrifugation speed of between 3000 and 10,000 rpm, preferentially greater than 5000 rpm and less than or equal to 10,000 rpm, advantageously between 5200 and 7500 rpm, in a particularly preferred manner between 6000 and 7000 rpm, and/or
- a purified aqueous liquid plant maceration flow of between 5 and 15 m³/h, preferably between 7 and 13 m³/h, preferentially between 8 and 12 m³/h, advantageously between 9 and 11 m³/h, in a preferred manner of about 10 m³/h, advantageously both.

6. The method according to one of the preceding claims, wherein said step c) comprises at least two pressing operations, preferably realised in succession.

7. The method according to the preceding claim, wherein said at least two pressing operations on said aqueous plant maceration are realised in succession, and wherein the pressure exerted on said aqueous plant maceration during the second pressing operation is exerted along a different axis from the axis along which the pressure is exerted on the aqueous plant maceration during the first pressing operation.

8. The method according to one of the preceding claims, wherein step b) is realised at a temperature of 20°C to 80°C, preferably 30°C to 75°C, preferentially 40°C to 70°C, advantageously 55°C to 65°C.

9. The method according to one of the preceding claims, comprising, before step b), at least one step of preprocessing said plant substrate, such as at least one plant substrate drying and/or size-reduction step, advantageously both.

10. The method according to one of claims 1 to 9, said maceration being performed in at least one maceration tank (1a, 1b) comprising therein, advantageously on its internal wall, at least three distinct levels of ultrasonic transducer(s), each level of ultrasonic transducer(s) comprising at least one, and preferably at least two, ultrasonic transducers.

11. A method for packaging an aqueous liquid plant maceration in a container for pharmaceutical, cosmetic, nutritional or veterinary use, such as a bottle, a single-dose sachet, a single-dose stick, a vial, a single-dose ampoule bottle or an ampoule, wherein said aqueous liquid plant maceration is the aqueous liquid plant maceration obtained in step c) or in step e), when step e) is present, of the method according to one of the preceding claims, said packaging method comprising the following step(s):
- if applicable, opening the container if it is sealed,
- filling, by any means, said container totally or partially using said aqueous liquid plant maceration then, if applicable,
- sealing said container filled in this manner, preferably hermetically.

12. A device for obtaining an aqueous liquid plant maceration, suitable for implementing the method according to one of claims 1 to 10, said device comprising:
i) at least one maceration tank (1a, 1b) comprising therein, advantageously on its internal wall, at least two distinct levels of ultrasonic transducer(s), each level of ultrasonic transducer(s) comprising at least one, and preferably at least two, ultrasonic transducers,
said at least one maceration tank (1a, 1b) further comprising at least one mechanical stirring means suitable for keeping the plant substrate at a predetermined distance from the ultrasonic transducers, said at least one maceration tank (1a, 1b) being suitable for receiving the plant substrate, the water and performing step b),
ii) at least one pressing element (2), such as at least one press (2), suitable for receiving, at the end of step b), the aqueous plant maceration and performing step c),
iii) preferably at least one centrifuge (3), preferentially a plate centrifuge (3), suitable for receiving the aqueous liquid plant maceration obtained in step c) and performing step d), if the method comprises step d),
iv) advantageously at least one turbidimeter, suitable for monitoring the turbidity of the aqueous liquid plant maceration, preferentially obtained in step d), if the method comprises step d).

13. The device according to claim 12, wherein said at least one maceration tank (1a, 1b) comprises therein, advantageously on its internal wall, at least three distinct levels of ultrasonic transducer(s); each level of ultrasonic transducer(s) comprising at least one, and preferably at least two, ultrasonic transducers.

14. The device according to claim 12 or 13, wherein said at least one maceration tank (1a, 1b), said at least one pressing element (2), said at least one centrifuge (3) - if present - and said at least one turbidimeter - if present- are connected to one another by a system of pipes.

15. The device according to one of claims 12 to 14, said device being automated.
